(19) Europäisches Patentamt
European Patent Office
Office européen des brevets

(11) Veröffentlichungsnummer: **0 009 851**
**B1**

(12) # EUROPÄISCHE PATENTSCHRIFT

(45) Veröffentlichungstag der Patentschrift: **20.01.82**

(51) Int. Cl.³: **C 07 C 179/047**

(21) Anmeldenummer: **79200561.3**

(22) Anmeldetag: **03.10.79**

(54) **Verfahren zur Herstellung von araliphatischen Dihydroperoxiden.**

(30) Priorität: **09.10.78 DE 2843957**

(43) Veröffentlichungstag der Anmeldung: **16.04.80 Patentblatt 80/8**

(45) Bekanntmachung des Hinweises auf die Patenterteilung: **20.01.82 Patentblatt 82/3**

(84) Benannte Vertragsstaaten: **BE CH DE FR GB IT LU NL SE**

(56) Entgegenhaltungen:
**DE-A-1 237 118**
**DE-A-2 352 580**

(73) Patentinhaber: **CHEMISCHE WERKE HÜLS AG, Postfach 1320, D-4370 Marl 1 (DE)**

(72) Erfinder: **Voges, Heinz Werner, Dr., Martin-Luther-Strasse 123, D-4270 Dorsten 21 (DE)**

(74) Vertreter: **Steil, Hanna, Dipl.-Chem. et al, RSP PATENTE-PB 40 Holsterhauser Strasse 160 Postfach 2840, D-4690 Herne 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 009 851 B1

# Verfahren zur Herstellung von araliphatischen Dihydroperoxiden

Nach der Erfindung wird die Kontamination eines zwangsläufig bei der Herstellung von Dihydroperoxiden anfallenden Abwasserstromes von darin enthaltenen hydroperoxidischen Bestandteilen vermindert. Aus den hydroperoxidischen Bestandteilen können sich äusserst unerwünschte phenolische Körper bilden.

Es ist bekannt, di-tert.-alkylsubstituierte aromatische Kohlenwasserstoffe der allgemeinen Formel

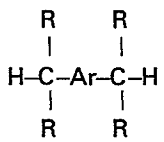

worin Ar ein aromatischer Rest und R eine Alkylgruppe bedeuten, insbesondere m- und/oder p-Diisopropylbenzol mit Sauerstoff oder sauerstoffhaltigen Gasen bei erhöhten Temperaturen, z.B. im Temperaturbereich von etwa 60 bis 120 °C zu den entsprechenden Dihydroperoxiden

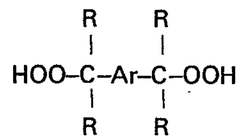

zu oxidieren.

Die wichtigsten, unter Oxidationsbedingungen ablaufenden chemischen Reaktionen des m- und/oder p-Diisopropylbenzols (DIPB) sind gemäss dem folgenden Reaktionsschema

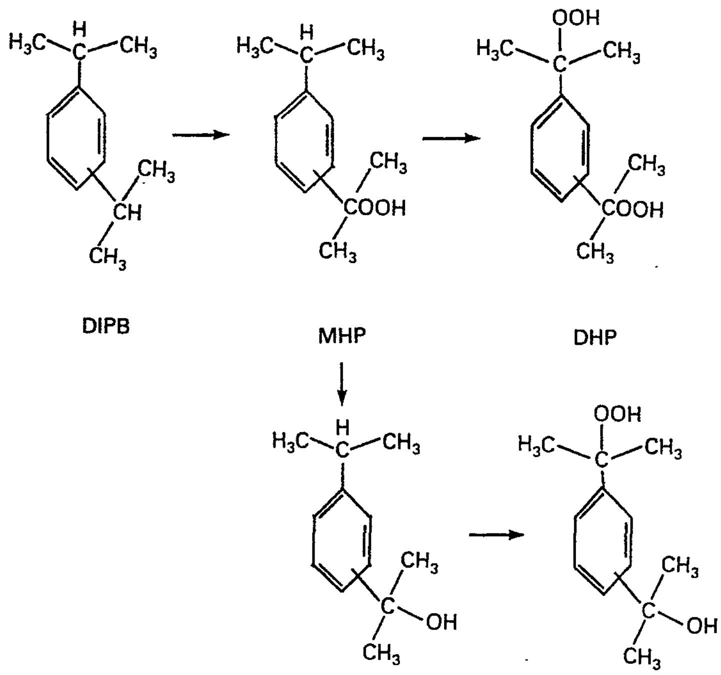

DIPB MHP DHP

MC HHP

die Oxidation des Kohlenwasserstoffs zum Monohydroperoxid MHP, die Weiteroxidation des MHP zum Dihydroperoxid DHP, die thermische und/oder katalytische Zersetzung des MHP zu dem Monocarbinol MC und dessen Weiteroxidation zu dem Hydroxyhydroperoxid HHP. Ziel eines technischen Verfahrens der Oxidation von di-tert. alkylierten Aromaten ist es im allgemeinen, die Dihydroperoxide aus dem Oxidationsgemisch zu isolieren und sie dann der bekannten, durch Mineralsäuren katalysierten Spaltung zu den entsprechenden phenolischen Substanzen zu unterwerfen. Im Falle von DIPB führte die Spaltung zu Hydrochinon und/oder Resorcin. Die Oxidation des Kohlenwasserstoffes, z.B. des m- und/oder p-DIPB wird im allgemeinen, u.a. aus Sicherheitsgründen, nur bis zu einem bestimmten Teilumsatz durchgeführt. Das Oxidationsgemisch enthält dann neben unumgesetztem Kohlenwasserstoff eine relativ grosse Menge des kinetischen Zwischenproduktes MHP und eine relativ kleine Menge an DHP und HHP.

Es ist seit langem bekannt, dass man das DHP und das HHP aus dem Oxidationsgemisch durch eine Extraktion desselben mit 1–12%iger wässriger Alkalilauge selektiv extrahieren kann. Als Raffinat hinterbleibt bei diesem Extraktionsvorgang ein Gemisch aus unumgesetztem Kohlenwasserstoff, der z.B. DIPB, dem Zwischenprodukt MHP und in stark vermindertem Masse auch noch DHP und HHP. Daneben geraten in dieses organische Gemisch aber auch gewisse Anteile an freier Alka-

lilauge hinein, z.B. in gelöster oder emulgierter Form. Führt man nun – wie es in einem technischen Verfahren selbstverständlich ist – das an DHP verarmte Gemisch erneut zu der Oxidationszone zurück, um weiteres DHP zu produzieren, so gerät mit dem Rückführgut auch freie Alkalilauge in den Oxidationsraum. Es ist aber bekannt, dass die hydroperoxidbildende Oxidation von araliphatischen Kohlenwasserstoffen am vorteilhaftesten, d.h. mit grösstmöglicher Ausbeute an Hydroperoxid, im schwach sauren bis schwach alkalischen Bereich stattfinden muss. Die nach der Extraktion im Rückführgut verbleibende Menge an Alkalihydroxid würde aber ohne Nachbehandlung stark alkalische Bedingungen in der Oxidation einstellen. In der DE-PS 12 37 118 ist deshalb vorgeschlagen worden, das im Rückführgut enthaltene Alkalihydroxid durch eine Behandlung mit gasförmigen $CO_2$ zu neutralisieren und das dadurch gebildete Alkalicarbonat bzw. Alkalibicarbonat durch eine Wasserwäsche zu entfernen.

Jedoch ist diese Verfahrensweise mit einem Nachteil verbunden. Verfährt man so, wie in der genannten Patentschrift beschrieben, so stellt man fest, dass das zur Wäsche eingesetzte Wasser neben den anorganischen Salzen auch gewisse Anteile an Hydroperoxiden aus dem Rückführgut herausgelöst. Das Waschwasser kann dann einen Hydroperoxidgehalt von 1–3 Gew.-% aufweisen. Es ist evident, dass ein solches Wasser nicht unbedenklich anderen Abwasserströmen einer chemischen Fabrik beigemischt werden kann, da aus den Hydroperoxiden äusserst unerwünschte phenolische Körper gebildet werden können. Des weiteren stellt man bei einer analytischen Untersuchung der ins Abwasser geratenen Hydroperoxide fest, dass diese zu 85–95% aus dem wertvollen Zwischenprodukt MHP, zu 4–10% aus HHP und zu 1–5% aus DHP bestehen.

Die Preisgabe des MHP-Bestandteiles in dem nach DE-PS 12 37 118 anfallenden Abwasser zieht eine Ausbeuteminderung im gesamten Prozess der Dihydroperoxid-Herstellung, der im fogenden noch im einzelnen beschrieben wird, von immerhin 1–3%, bezogen auf das eingesetzte DIPB, nach sich.

Somit stellte sich die Aufgabe, bei der Herstellung von Dihydroperoxiden nach Massnahmen zu suchen, welche die Kontamination des Abwassers mit Hydroperoxiden entscheidend vermindert und gleichzeitig die Gesamtausbeute an Dihydroperoxiden erhöhen.

Die Aufgabe wurde dadurch gelöst, dass die Hydroperoxide mit aliphatischen oder aromatischen Kohlenwasserstoffen, z.B. mit den n- und iso-Paraffinkohlenwasserstoffen im C-Zahl-Bereich von 5–8, mit Benzol, Toluol, Äthylbenzol, Cumol und anderen alkylierten Aromaten oder mit Gemischen solcher Kohlenwasserstoffe aus dem Waschwasser bei gewöhnlicher oder erhöhter Temperatur, z.B. im Temperaturbereich von 20–60 °C, extrahiert werden.

Gegenstand der Erfindung ist daher ein Verfahren von araliphatischen Dihydroperoxiden der allgemeinen Formel

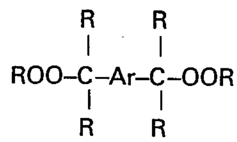

worin Ar = aromatischer Rest und R = H oder $C_1$–$C_4$-Alkyl bedeuten, durch Oxidation von Kohlenwasserstoffen der Formel

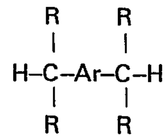

mit Sauerstoff oder sauerstoffhaltigen Gasen, Extraktion der Dihydroperoxide mit 1–12%ger Alkalilauge sowie anschliessender Neutralisation des im organischen Raffinat verbliebenen Alkalihydroxids mit $CO_2$, gefolgt von einer Auswaschung der hierbei gebildeten Alkalicarbonate mit Wasser, welches dadurch gekennzeichnet ist, dass das zur Alkalicarbonat-Auswaschung benutzte Waschwasser vor der Verwerfung durch Extraktion mit einem Kohlenwasserstoff von gelösten hydroperoxidischen Bestandteilen befreit wird, und die so extrahierten hydroperoxidischen Bestandteile gegebenenfalls in den Prozess rückgeführt werden.

Alle erfindungsgemäss als Extraktionsmittel einsetzbaren Kohlenwasserstoffe sind selbst nur in zu vernachlässigendem Masse in dem Abwasser löslich. Die Wahl des zur Abwasserextraktion eingesetzten Kohlenwasserstoffes trifft man zweckmässigerweise so, dass sich aus dem Extrakt möglichst einfach sowohl das Lösungmittel wie auch die Hydroperoxide zurückgewinnen lassen. Ein mögliches Trennverfahren ist die Destillation des Lösungsmittels, wobei das Hydroperoxidgemisch als Sumpfprodukt anfällt. Mit Vorteil führt man diese Lösungsmitteldestillation in einem Dünnschichtverdampfer durch. Da Hydroperoxide sich allgemein bei Temperaturen oberhalb von etwa 100 °C thermisch rasch zu zersetzen beginnen, setzt man bei der erfindungsgemässen Extraktion entweder einen Kohlenwasserstoff mit niedrigem Siedepunkt ein oder führt – falls ein hochsiedendes Extraktionsmittel bevorzugt wird – die Destillation am Dünnschichtverdampfer bei vermindertem Druck durch.

Der Wert der Erfindung tritt besonders klar hervor, wenn als Extraktionsmittel der in die Oxidation eingesetzte di-tert. alkylsubstituierte Aromat gewählt wird. In diesem Fall wird der Extrakt ohne zusätzliche Aufarbeitungsstufe mit dem Rückführgut vereinigt und in den Prozess zurückgeführt.

Die erfindungsgemässe Extraktion der Hydroperoxide aus dem Waschwasser des Oxidations-Rückführgutes kann mit den gebräuchlichen Extraktionsvorrichtungen, z.B. Mixer-Settler-Apparaturen durchgeführt werden. Meist genügt bereits ein einmaliges Inkontaktbringen des Waschwassers mit dem kohlenwasserstoffartigen Extraktionsmittel mit anschliessendem Wiederabsitzenlassen der Phasen, um die Hydroperoxide weitgehend in das Extraktionsmittel zu überführen. Beispielsweise wird der Hydroperoxidgehalt im Waschwasser von 1–3 Gew.-% auf 0,1–0,3 Gew.-%, d.h. auf 1/10 seines ursprünglichen Wertes herabsenkt. Ein wässriger Strom mit derart niedrigem Resthydroperoxidgehalt kann unbedenklich in den Abwasserkanal gegeben werden.

In die erfindungsgemässe Extraktion werden Waschwasser und Kohlenwasserstoff im Gewichtsverhältnis von etwa 1:1 bis zu etwa 10:1 eingesetzt.

Zwar ist es zum Zwecke der möglichst vollständigen Entfernung der Hydroperoxide aus dem Waschwasser günstig, wenn relativ viel Extraktionsmittel eingesetzt wird. Dem steht in der technischen Praxis aber entgegen, dass dann auch eine grosse Menge an Extraktionsmittel unter relativ grossem Kostenaufwand zurückgewonnen werden muss. Im besonderen setzt man Waschwasser und Extraktionsmittel daher im Verhältnis von etwa 3:1 bis 6:1 ein.

Wählt man für die erfindungsgemässe Extraktion den zur Dihydroperoxid bildenden Oxidation eingesetzten Kohlenwasserstoff selbst, so gelingt es ohne weiteres, die Hydroperoxide aus dem Waschwasser gerade mit der Menge an Kohlenwasserstoff zu extrahieren, die notwendigerweise im technischen Kreisprozess ohnehin zugeführt werden muss, um die Menge an produziertem Dihydroperoxid molmässig zu ersetzen. Mit anderen Worten: In der bevorzugten Ausführungsform der Erfindung kann der Einsatz-Kohlenwasserstoff selbst zur Waschwasser-Extraktion eingesetzt werden, ohne dass die Molbalance des technischen Kreisprozesses gestört wird.

Das beigefügte Blockschema 1 veranschaulicht die Erfindung in ihrer allgemeinen Ausführungsform, das Blockschema 2 verdeutlicht die Erfindung in der bevorzugten Ausführungsform am Beispiel der Dihydroperoxidherstellung aus Diisopropylbenzolen.

Blockschema 1

Di-tert. alkylsubstituierter aromatischer Kohlenwasserstoff wird in dem Oxidationsreaktor A mit Sauerstoff oder sauerstoffhaltigem Gas bis zu einem gewünschten Teilumsatz oxidiert. Das jetzt dihydroperoxidhaltige Oxidat wird über Leitung 1 der Extraktionseinheit B zugeführt, in der es von der über Leitung 2 zufliessenden 1–12%igen Alkalilauge extrahiert wird. Während jetzt dihydroperoxid-beladene Lauge über Leitung 4 abfliesst, strömt das Rückführgut über Leitung 3 in die Neutralisations- und Wascheinheit C. Durch $CO_2$ aus Leitung 5 wird das mitgeschleppte Alkalihydroxid im Rückführgut neutralisiert, durch Wasser aus Leitung 6 werden die Carbonate ausgewaschen. Das Rückführgut fliesst dann über Leitung 7 in den Oxidationsreaktor zurück. Das Waschwasser, das ausser den Alkalicarbonaten durch Hydroperoxide aus dem Rückführgut aufgenommen hat, fliesst über Leitung 8 in die Extraktionseinheit D. Hier findet die erfindungsgemässe Extraktion der Hydroperoxide durch den über Leitung 9 zufliessenden Kohlenwasserstoff statt, indem das Waschwasser zunächst innig mit dem Kohlenwasserstoff vermischt wird und das Gemisch dann wieder in die Phasen getrennt wird. Über Leitung 10 wird jetzt ein Waschwasser abgezogen, das nur noch 0,1–0,3 Gew.-% Hydroperoxid enthält.

Über Leitung 11 wird das jetzt mit Hydroperoxid beladene Extraktionsmittel der Trenneinheit, z.D. der Destillationseinheit E zugeführt. Hier wird das Extraktionsmittel abgezogen und über Leitung 9 wieder der Extraktionseinheit D zugeführt, nachdem gewisse, im technischen Betrieb unvermeidbare Verluste an Kohlenwasserstoff über Leitung 13 ergänzt werden. Die in Einheit E zurückgewonnen Hydroperoxide werden über Leitung 12 mit dem Rückführgut vereinigt. Die frisch zur Oxidation gelangende Menge an tert.-alkyl-substituiertem aromatischem Kohlenwasserstoff wird über Strom 14 ebenfalls dem Rückführgut beigemischt oder auch direkt in den Oxidationsreaktor eingespeist.

Blockschema 2

Das Oxidat des m- oder/und p-DIPB verlässt über Leitung 1 den Oxidationsreaktor A und fliesst der Extraktionseinheit B zu. Hier findet die Extraktion des DHP und des HHP durch die aus Leitung 2 zufliessende 1–12%ige wässrige Alkalilauge statt. Die mit DHP, HHP und nur sehr wenig MHP beladene Extraktionslauge fliesst der Extraktionseinheit E zu. Das an DHP und HHP verarmte Rückführgut wird über Leitung 3 in die Neutralisations- und Wascheinheit C überführt. Aus Leitung 5 strömt $CO_2$ zur Neutralisation der mitgerissenen Alkalilauge, aus Leitung 6 strömt $H_2O$ zur Auswaschung der Alkalicarbonate in die Einheit C. Das von Carbonaten freigewaschene Rückführgut fliesst über Leitung 7 wieder in den Oxidationsreaktor A zurück. Das Waschwasser aus Leitung 6, nunmehr beladen mit Alkalicarbonaten und etwa 1,1 Gew.-% Hydroperoxiden fliesst über Leitung 8 in die erfindungsmässige Extraktionsstufe D. Hier wird diese Wasserphase mit dem aus Leitung 9 zugeführte DIPB in einer Mischzone vermischt. Die Waschwassermenge, die über Leitung 6 zufliesst, wird so bemessen, dass die zu dem über Leitung 9 zufliessenden DIPB im Gewichtsverhältnis 4,5 zu 1 steht.

Nach Durchlaufen einer Absitzzone werden die wieder getrennten Phasen über zwei Leitungen abgezogen. Das Abwasser wird über Leitung 10 entnommen, es enthält 0,17 Gew.-% Hydroperoxid und wird dem Abwasserkanal zugeführt. Die DIPB-Phase, die jetzt mit etwa 4,2 Gew.-% Hydroperoxid beladen ist, wovon 88% MHP ist, fliesst über Leitung 11 in die Extraktionseinheit E, der auch der mit DHP und HHP beladene Alkalistrom über Leitung 4 zugeflossen ist. Durch Vermischen und Wiederabsitzenlassen beider Ströme in E wird bewirkt, dass die DIPB-Phase an die Alkaliphase ihren kleinen DHP- und HHP-Anteil abgibt und andererseits aus der Alkalilauge einen mitgeführten MHP-Anteil aufnimmt. Die Alkaliphase verlässt, von MHP befreit, die Einheit E über Leitung 13 zur weiteren Verarbeitung. Die DIPB-Phase wird über Leitung 12 mit dem Rückführgutstrom 3 vereinigt. Die mit Leitung 9 eingeführte DIPB-Menge wird so bemessen, dass sie molmässig – gerade der Summe der in Leitung 13 abfliessenden Mole DHP und HHP entspricht.

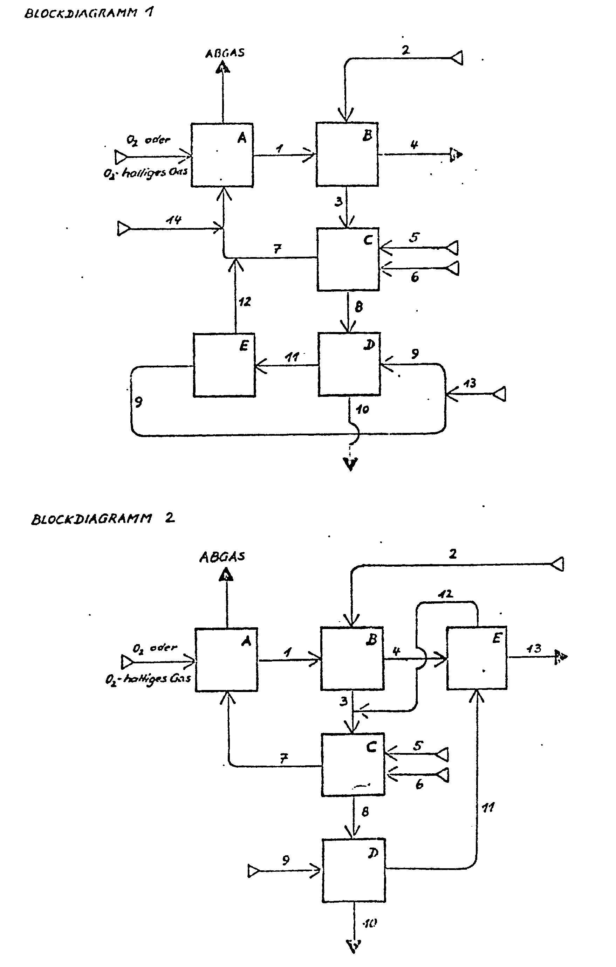

**Patentansprüche**

1. Verfahren zur Herstellung von araliphatischen Dihydroperoxiden der allgemeinen Formel

R R<br>
| |<br>
ROO–C–Ar–C–OOR<br>
| |<br>
R R

worin Ar = aromatischer Rest und R = H oder $C_1$–$C_4$-Alkyl bedeuten, durch Oxidation von Kohlenwasserstoffen der Formel

R R<br>
| |<br>
H–C–Ar–C–H<br>
| |<br>
R R

mit Sauerstoff oder sauerstoffhaltigen Gasen, Extraktion der Dihydroperoxide mit 1–12%iger Alkalilauge sowie anschliessender Neutralisation des im organischen Raffinat verbliebenen Alkalihydroxids mit $CO_2$, gefolgt von einer Auswaschung der hierbei gebildeten Alkalicarbonate mit Wasser, dadurch gekennzeichnet, dass das zur Alkalicarbonat-Auswaschung benutzte Waschwasser vor der Verwerfung durch Extraktion mit einem Kohlenwasserstoff von gelösten hydroperoxidischen Bestandteilen befreit wird und die so extrahierten hydroperoxidischen Bestandteilen gegebenenfalls in den Prozess rückgeführt werden.

2. Verfahren nach Anspruch 1, dadurch gekennzeichnet, dass man für die Extraktion des Waschwassers einen Kohlenwasserstoff aus der Gruppe der Aliphaten von $C_5$ bis $C_8$ oder aus der Gruppe der Aromaten Benzol, Toluol, Äthylbenzol, Cumol oder der disubstituierten Aromaten verwendet.

3. Verfahren nach den Ansprüchen 1 und 2, dadurch gekennzeichnet, dass man aus dem hydroperoxidischen Extrakt das Lösungsmittel abtrennt, es für die Extraktion des Abwassers wiederverwendet und die dabei zurückgewonnenen Hydroperoxide in den Prozess zurückführt.

4. Verfahren nach Anspruch 1, dadurch gekennzeichnet, dass als Extraktionsmittel für das Waschwasser der zur dihydroperoxidbildenden Oxidation eingesetzte Kohlenwasserstoff verwendet wird und der gesamte hydroperoxidhaltige Abwasserextrakt in den Prozess zurückgeführt wird.

5. Verfahren nach den Ansprüchen 1 und 4, dadurch gekennzeichnet, dass der Kohlenwasserstoff in der Menge zur Abwasserextraktion eingesetzt wird, die molmässig der Produktion an Dihydroperoxid entspricht.

6. Verfahren nach den Ansprüchen 1, 4 und 5, dadurch gekennzeichnet, dass zur Oxidation und Waschwasserextraktion p-Diisopropylbenzol eingesetzt wird.

7. Verfahren nach den Ansprüchen 1, 4 und 5, dadurch gekennzeichnet, dass zur Oxidation und Waschwasserextraktion m-Diisopropylbenzol eingesetzt wird.

8. Verfahren nach den Ansprüchen 1, 4 und 5, dadurch gekennzeichnet, dass für Oxidation und Waschwasserextraktion ein Gemisch aus m- und p-Diisopropylbenzol eingesetzt wird.

**Revendications**

1. Procédé pour la fabrication de dihydroperoxydes araliphatiques de formule générale:

R R<br>
| |<br>
ROO–C–Ar–C–OOR<br>
| |<br>
R R

(dans laquelle Ar est un reste aromatique et R = H ou bien un alkyle en $C_1$–$C_4$) par oxydation d'hydrocarbures de formule:

R R<br>
| |<br>
H–C–Ar–C–H<br>
| |<br>
R R

avec de l'oxygène ou un gaz contenant de l'oxygène, puis extraction du dihydroperoxyde avec une lessive alcaline à 1 à 12% ainsi qu'une neutralisation de l'hydroxyde alcalin restant dans le raffinat organique avec $CO_2$, suivi par un enlèvement, par lavage à l'eau, du carbonate alcalin ainsi formé, procédé caractérisé en ce que l'eau de lavage utilisée pour la séparation par lavage du carbonate alcalin est, avant son évacution comme eau usée, libérée de ses constituants hydroperoxydes en dissolution, par extraction au moyen d'un hydrocarbure, et les composants hydroperoxydiques ainsi recupérés sont ramenés dans le processus de fabrication.

2. Procédé suivant la revendication 1, caractérisé en ce que, pour le traitement d'extraction de l'eau de lavage, on utilise un hydrocarbure du groupe des aliphatiques de $C_5$ à $C_8$ ou du group des aromatiques benzène, toluène, éthyl-benzène, cumène ou aromates disubstitués.

3. Procédé suivant l'une des revendications 1 et 2, caractérisé en ce qu'on sépare le solvant de l'extrait hydroperoxydique, et on l'utilise à nouveau pour le traitement d'extraction de l'eau usée, l'hydroperoxyde ainsi récupéré étant ramené dans le circuit du processus de fabrication.

4. Procédé suivant la revendication 1, caractérisé en ce que, comm moyen d'extraction pour l'eau de lavage, on utilise l'hydrocarbure qui a été mis en œuvre pour l'oxydation formant le dihydroperoxyde el la totalité de l'extrait d'eau usée contenant de l'hydroperoxyde est ramenée dans le circuit du processus.

5. Procédé suivant l'une quelconque des revendications 1 et 4, caractérisé en ce que l'hydrocarbure est mis en œuvre pour le traitement d'extraction de l'eau usée, en une quantité molaire qui correspond à la production du dihydroperoxyde.

6. Procédé suivant l'une quelconque des revendications 1, 4 et 5, caractérisé en ce que, pour l'oxydation ainsi que pour l'extraction de l'eau lavage, on utilise le p-diisopropyl-benzène.

7. Procédé suivant l'une quelconque des revendications 1, 4 et 5, caractérisé en ce que pour l'oxydation ainsi que pour l'extraction de l'eau de lavage, on utilise le m-diisopropyl-benzène.

8. Procédé suivant l'une quelconque des revendications 1, 4 et 5, caractérisé en ce que pour l'oxydation ainsi que pour l'extraction de l'eau de lavage, on utilise un mélange de m- et p-diisopropyl-benzène.

**Claims**

1. A method for the preparation of araliphatic dihydroperoxides of the general formula:

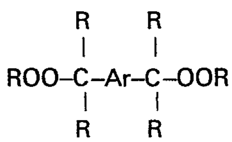

wherein Ar = aromatic radical and R = H or a $C_1$–$C_4$ alkyl by oxidation of a hydrocarbon of the formula:

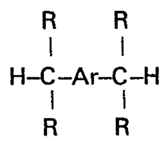

with oxygen or an oxygen-containing gas, extraction of dihydroperoxides with 1–12% alkali metal lye as well as following neutralization of the alkali metal hydroxide remaining in the organic raffinate with $CO_2$ followed by a washing of the thus formed alkali metal carbonate with water, wherein the wash water utilized for the washing of alkali metal carbonate is, prior to discarding, extracted of any dissolved hydroperoxide components with a hydrocarbon and the so extracted hydroperoxide components are optionally recycled into the process.

2. A method according to Claim 1, wherein for the extraction of the wash-water one uses a hydrocarbon of the group of $C_5$–$C_8$ aliphatics or of the group of aromatics benzene, toluene, ethylbenzene, cumene or disubstituted aromatics.

3. A method according to Claims 1 and 2, wherein the solvent which is separated from the hydroperoxide extract, is the reutilized for the extraction of the wash-water and the thus recovered hydroperoxides are recycled into the process.

4. A method according to Claim 1, wherein as extraction medium for the wash-water the hydrocarbon is used being applied for the oxidation to from the dihydroperoxide, and the collected hydroperoxide-containing wash-water-extract is recycled into the process.

5. A method according to Claims 1 and 4, wherein the hydrocarbon is utilized for the extraction of the wasch-water in such an amount, that it corresponds to the molar production of dihydroperoxides.

6. A method according to Claims 1, 4 and 5, wherein p-diisopropylbenzene is utilized for the oxidation and wash-water extraction.

7. A method according to Claims 1, 4 and 5, wherein n-diisopropylbenzene is utilized for the oxidation and wash-water extraction.

8. A method according to Claims 1, 4 and 5, wherein a mixture of m- and p-diisopropylbenzene is utilized for the oxidation and wash-water extraction.

BLOCKDIAGRAMM 1

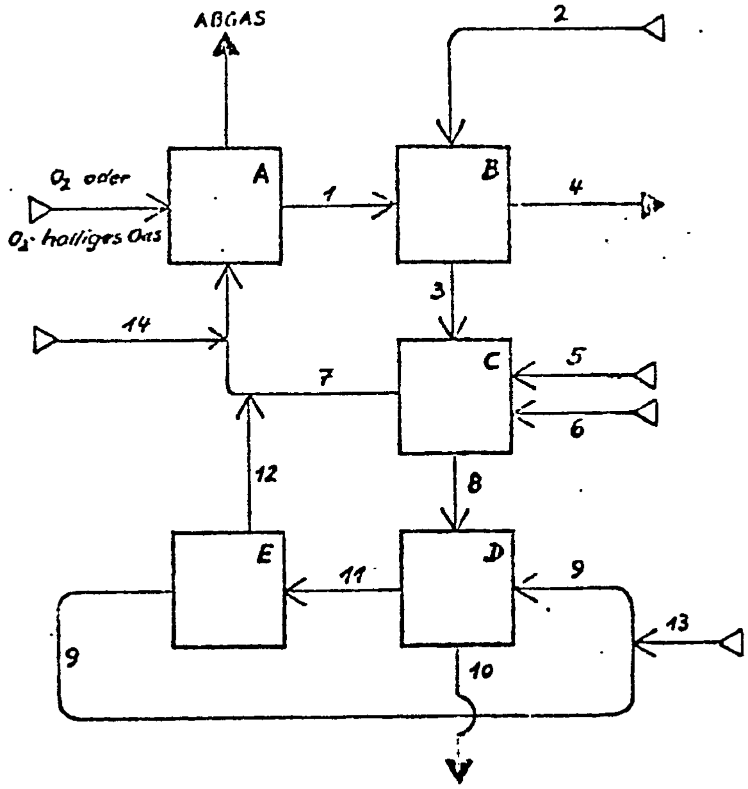


BLOCKDIAGRAMM 2